# EUROPEAN PATENT APPLICATION

(11) **EP 3 900 682 A1**
(43) Date of publication of application: **27.10.2021**
(21) Application number: 20382330.7
(22) Date of filing: 22.04.2020
(51) Int. Cl.: A61F 5/445

(54) **CONTINUITY DEVICE FOR DERIVATIVE ILEOSTOMY**

(71) Applicant: Servizo Galego de Saude, 15703 Santiago de Compostela (ES)
(72) Inventor: NOGUERA AGUILAR, José Francisco, 15006 A Coruña (ES); AGUIRREZABALAGA GONZÁLEZ, Javier Antonio, 15006 A Coruña (ES); CENTENO CORTES, Alberto, 15006 A Coruña (ES)
(74) Representative: Hoffmann Eitle

(57) **Abstract**

The present invention refers to a continuity device (1) to obtain preoperative predictive test to know if the results of the derivative ileostomy closure surgery will be as expected, and to predict the correct anorectal functioning after the digestive reconstruction surgery. In addition, the device also provides preoperative improvement of the efferent or distal loop. The device for derivative ileostomy comprises a collector tube (2) suitable to be inserted inside a first bowel segment (5) of an ileostomy, a distal tube (4) suitable to be inserted inside a second bowel segment (6) of an ileostomy, and a connector tube (3) having a curved configuration, and made of a deformable material. The connection tube can be connected and disconnected with the collector and distal tubes, such that, when the connector tube is connected fecal material can circulate through the collector, connection and distal tubes. First and second inflatable bags (8, 9) are respectively mounted on the collector tube and the distal tube.

## Description

### Field and object of the invention

The present invention generally relates to stomal medical devices for treating patients affected by colorectal cancer.

More specifically, an object of the invention is to provide a continuity device that enable to obtain preoperative predictive test to know if the results of the derivative ileostomy closure surgery will be as expected, and to predict the correct anorectal functioning after the digestive reconstruction surgery.

An additional object of the invention is to provide continuity device to prepare the efferent loop of the ileostomy for the reconstruction surgery, avoiding intestinal atrophy and improving the caliber of the loop for the subsequent ileoileal anastomosis, as well as the conditioning prior to bowel reconstruction to improve distal bowel capacity and function.

### Background of the invention

Colorectal cancer represents 9% of all cancers around the globe. It is the third most common cause of death in the USA. In 2016, 158,000 new cases of colorectal cancer were recorded. There are similar incidence rates for cancer of the colon in both sexes, and a slight male predominance for rectal cancer. In the UK, colorectal cancer is the second most common cause of death, with 20,000 new cases every year. In addition, in many countries around the world, including European countries, rates of colorectal cancer are high.

Rectal cancer shows a growing trend. Classically, it accounted for one third of patients born with colorectal cancer, but nowadays it is estimated to account for 40% of CRC and with a growing trend. Moreover, this cancer is affecting younger patients in a selective way, which means a greater interest in sphincter preservation and a good posterior functionality. This encourages the research in order to improve the quality of this process of improvement of the surgery of reestablishment of the colorectal continuity after a derivative ileosotomy.

In middle and low anterior resection for rectal cancer (66% of rectal cancer), protective ileostomy is strongly suggested when anastomosis is performed less than 8 - 10 cm from the anal verge or when a previous treatment with radiotherapy was administrated. The construction of a protective stoma significantly reduces the incidence of anastomotic leakage and re-operation but it may cause another related problems due to the bowel absence of transit and the surgical closure. In many patients the closure of this ileostomy is delayed for months due to postoperative treatments and the long waiting lists presented by the public health system.

Most of these patients are carriers of loop ileostomy because they have previously had a rectal resection with low colorectal anastomosis that usually leads to the so-called "anterior resection syndrome", with the possibility of postoperative anal incontinence and anorectal dysfunction, sometimes very difficult to solve.

The loop-ileostomy is a derivative stoma to prevent postoperative anastomotic problems in colorectal surgery. This preventive stoma is being performed more and more frequently due to the advancement in surgical rectal techniques. This surgical advancement makes possible to save the distal part of the rectum and the anus.

Decades ago the majority of stomas were colic (colostomies) while nowadays we are changing colic stomas for ileal stomas (ileostomy), whose function is to protect a colorectal anastomosis from high risk of dehiscence due to the proximity of the anorectal sphincter system.

Patients who are carriers of derivative ileostomies present a defunctionalization of the preserved rectum and the area where the colorectal anastomosis has been performed for several months, even years. This is due to post-operative treatments with systemic chemotherapy and long waiting lists in the public health system. The patient who has already been operated on for his or her early neoplasm and needs the closure of the ileostomy and transit reconstruction is no longer a priority for the system and is often "abandoned" with his or her stoma problem.

As a result of all these months of defunctionalization, at the time of stoma closure we do not know how the patient is doing from a functional point of view in relation to defecatory function and anorectal continence. It is possible that his continence is correct but it is possible that there is some incontinence and anorectal dysfunction that are unnoticed until the moment of reconstruction. These types of alterations are well defined and are known as "anterior rectal resection syndrome" and "ultra-low resection syndrome" and both refer to this type of anorectal dysfunction that alters rectal reservoir capacity, anorectal coordination and anal continence.

Therefore, one clinical problem after this waiting time is the absence of information for the surgeon and the patient about the rectoanal function. Previously to close the ileostomy and to restore the bowel continuity it would be desirable to know whether the patient will have any problem concerning the distal function of the rectum and anus.

The existence of a transit reconstruction simulation maneuver by simulating stoma closure would be of great help in predicting which patients may have problems after ileal transit reconstruction.

Furthermore, it would be desirable to made available preoperative techniques that improves safety of ileal anastomosis, with less incidence of complications (dehiscence, stenosis), when operating on a distal ileal segment improved with the preparation by distal instillation of solutions or drugs to improve the caliber for a safer ileal anastomosis, and improvement of the villous atrophy for a better intestinal absorption.

### Summary of the invention

The present invention is defined in the attached independent claim, and satisfactorily solves the drawbacks of the prior art, by providing a "U-ileostomy" medical device that comprises a tube-connection between the two parts of the loop ileostomy, such that, by using this medical device it is possible to check by clinical test at a pre-surgery stage, the recto anal function with a good prediction of the functional results of the surgery before the ileostomy's closure, such that optimal candidates can be identified and the right time for surgery, as well as the improvement of preoperative techniques of the efferent or distal loop.

In particular, the invention refers to a continuity device for derivative ileostomy, that comprises three connectable tubes, namely: a collector tube suitable to be inserted inside a first bowel segment of an ileostomy (proximal afferent limb), a distal tube suitable to be inserted inside a second bowel segment of an ileostomy (distal efferent limb), and a connector tube connectable and disconectable with the collector and distal tubes. Having a device formed by three tubes that can be easily connected and disconnected, provides the advantage the device is easy to install in a patient, and any of the tubes can be easily replaced or cleaned if it gets obstructed.

The connection tube has a curved or arcuated configuration, and it is made of a deformable material or alternatively it has a compressible configuration. In a preferred embodiment, the connection tube has a U-shaped or any other curved configuration suitable for this purpose.

The above-mentioned three tubes are configured such that the connection tube can be connected and disconnected with the collector and distal tubes, so that, when the three tubes are connected during a derivative ileostomy procedure, fecal material can circulate from the first bowel segment to the second bowel segment through the collector, connection and distal tubes. For that, the tubes are dimensioned in way that the three tubes can be coupled directly, or alternatively the device incorporates connection elements for connecting and disconnecting the tubes.

The device further comprises retention means configured to retain the collector and distal tubes fixed to the bowels segments while they are in use, avoiding displacement of the device. In a preferred embodiment, these retention means comprise a first and second inflatable bags respectively mounted on the collector tube and the distal tube, such that these inflatable bags are configured to retain the respective tubes fixed to a part of the bowel segments at which are inserted when they are inflated.

In addition, the device includes first and second conduits extending respectively along the collector and distal tubes, and communicating respectively with the interior of the first and second bags. These, first and second conduits are accessible from outside the device for inflating the bags, by means of any proper pumping means like for example, a syringe.

With the "U-ileostomy" medical device for ileal reconstruction of the invention, the condition of the defunctionalized bowel segments is improved prior to surgery. Thanks to the transit of the ileal content and the irrigations trough the device it is possible to improve and optimize the diameter of the distal ileum and its absorption's capacity.

The device allows for the improvement of the ileal loop and the colon and rectum that have been defunctionalized for several months. The lack of transit leads to villous atrophy and lack of initial absorption, as well as a thinning and collapse of the digestive tract. All this condition problems when performing the ileo-ileal anastomosis due to discrepancy between the calibers of the two intestinal segments, as well as absorption problems in the first days or weeks after the reconstruction of the digestive transit.

All this would mean an improvement in the care of patients with colorectal cancer who undergo loop derivative ileostomy as a temporary derivation mechanism to alleviate an obstructive condition or to protect an anastomosis with risk of dehiscence.

By using the "U-ileostomy" device, the absence of the stoma is simulated since the ileal continuity is artificially restored. In addition, as many clinical tests or reconstruction trials as necessary in the patient can be performed, and the test can be suspended at the time simply by removing the device.

With the "U-ileostomy" transit reconstruction simulation device, the condition of the dysfunctional bowel segments can be improved prior to surgery by optimizing the caliber of the distal ileum and absorption in the colon. The device is safe for the patient and easy to use. It does not pose an additional problem to stoma management as it is inserted into the ileal segments and covered by the ileostomy bag.

### Brief description of the drawings

Preferred embodiments of the invention are henceforth described with reference to the accompanying drawings, wherein:
Figures 1 and 3.- shows schematic representations of the device of the invention, wherein arrows indicate the rectal material flow direction.
Figure 2.- shows a perspective view partially sectioned of a practical implementation of the invention.

### Preferred embodiment of the invention

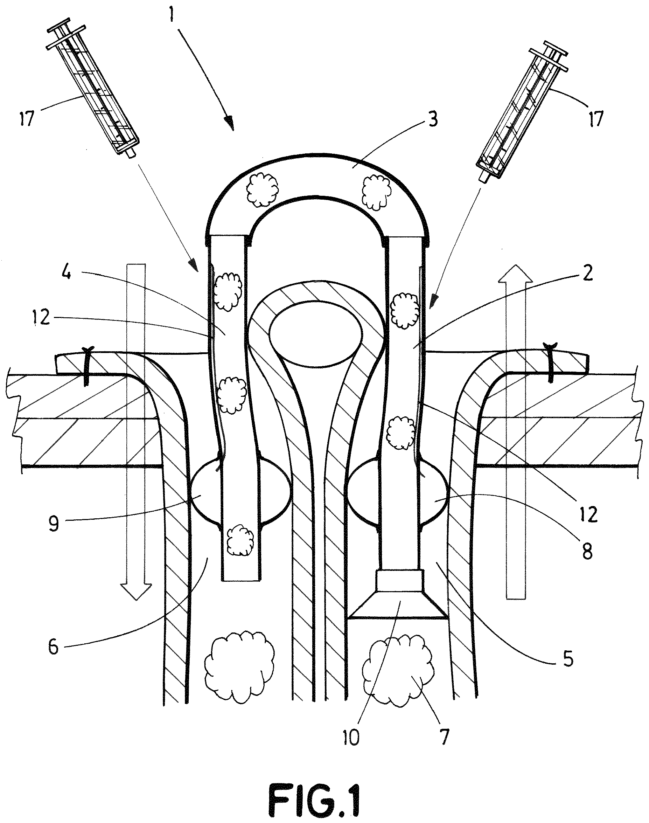

**Figure 1** shows a schematic representation of the "U-ileostomy" device (1) of the invention, that comprises a collector tube (2) suitable to be inserted inside a first bowel segment (5) of an ileostomy, a distal tube (4) suitable to be inserted inside a second bowel segment (6) of an ileostomy, and a connector tube (3) having a U-shaped configuration, and having two ends respectively coupled with a proximal end of the collector and distal tubes (2,4).

The connector tube ((3) has a U-shaped configuration in this implementation, and it is made of silicone, such that this tube can be compressed manually by a surgeon in order to force fecal material (7) to flow towards the distal tube (4).

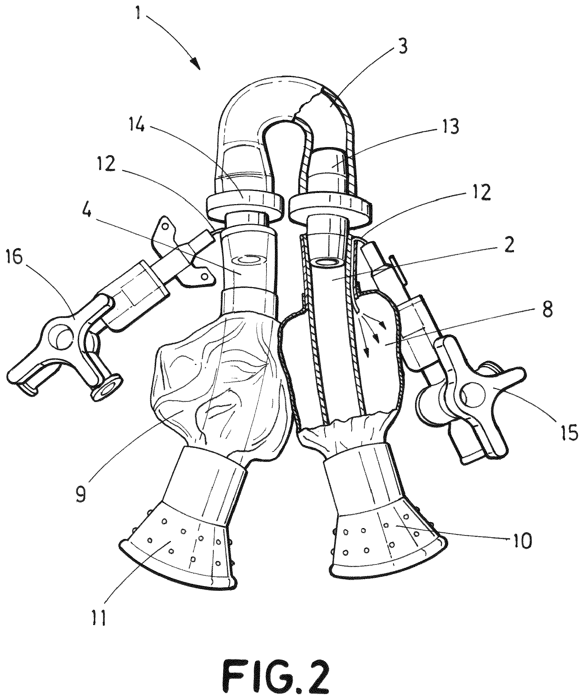

As shown in **Figure 2****,** for the connection of the three tubes, the device (1) is provided with a pair of connection elements (13), which consist of tubular bodies having one end connectable with one end of the connector tube (3) and another end connectable with a proximal end of the collector tube (2) or a proximal end of the distal tube (4). Each connection element (13) has a gripping wall (14) transversally arranged and placed between the two ends of the connection element (13), which facilities pulling of the connection element (13) to disconnect the tubes.

The connection elements (13) facilitate manual coupling and decoupling between the tubes, so as to allow an easy replacement of any of the tubes or cleaning operation, if any of the tubes get obstructed.

The device (1) further comprises first and second inflatable bags (8,9) respectively mounted on the collector tube (2) and the distal tube (4). The inflatable bags (8,9) are configured to retain the respective tubes fixed to a part of the bowel segments at which are inserted, by pressing internally on the bowels internal surface, avoiding any damage to the bowel tissue. Preferably, the inflatable bags (8,9) are made of silicone.

For inflating the bags (8,9), the device (1) incorporates first and second conduits (12) extending respectively along the collector and distal tubes (2,4) as shown in Figure 2. These conduits communicate respectively with the interior of the first and second bags (8,9) with the exterior of the device, such that, first and second conduits are accessible from outside the device (1) for inflating the bags. For this purpose, the device (1) incorporates first and second conventional valves (15,16) externally placed to the device, and connected respectively with the first and second conduits (12). Each of these valves (15,16) has an opening for the connection of a syringe (17) as it is schematically represented in Figure 1.

The first and second conduits (12) are joined respectively to an inner surface of the collection and distal tubes (2,4), or alternatively they are integrally formed with the collection and distal tubes.

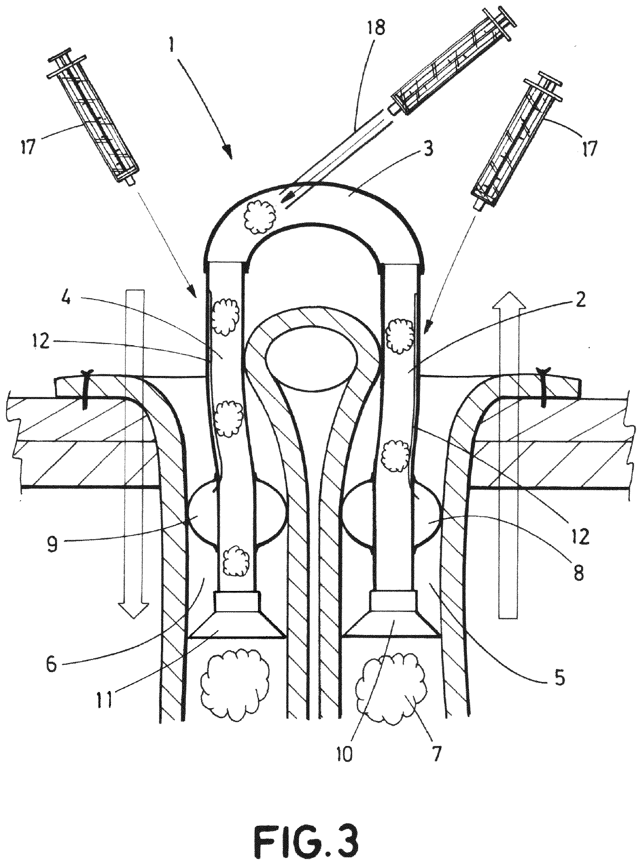

In the preferred embodiment of **Figure 3****,** the device (1) includes a third conduit (18) fluidly communicated with the interior of the connection tube (3) interior, and accessible from outside the connection tube (3). This third conduit (18) can be used as irrigation tube to inject washing liquids to clean the connector and distal tubes (2,4), and to inject chemical substances into the connection tube (3) towards the second bowel segment (6). Also in this embodiment, a second funnel (11) is fitted at the distal end of the distal tube (4), in order to prevent longitudinal displacements of the distal (4).

The collection and the distal tubes (2,4) are made of a flexible material, and their distal end has a funnel configuration. Preferably and as shown in Figure 1, a first frusto-conical funnel member (10) is coupled at a distal end of the collection tube (2), and optionally a second frusto-conical funnel (11) is coupled at a distal end of the distal tube (4). Both funnels (10,11) are preferably made of a silicone material, and are dimensioned to fit at the bowel segment to collect all fecal material (/ileal content) (7) into the collection tube (2), and to prevent this material from flowing between the collection and the bowel, thereby preventing that that ileal content could exert pressure on the bags (8,9) and displace the collection tube from the correct position.

As represented more clearly in **Figure 2****,** a part of each inflatable bag (8,9) is retained between the collection or distal tube (2,4) and a part of a frusto-conical funnel (10,11).

The medical device is manufactured with biocompatible and eco-friendly materials, latex-free and easy to clean and to sterilize.

Therefore, with the above described structure of the "U-ileostomy" device of the invention, at least the following advantages and functionalities are accomplished:
- the device is safe for the patient and easy to use,
- it allows checking with a clinical test the later rectoanal function with a good prediction of the functional results of the surgery before the ileostomy's closure,
- it is not an additional problem to the management of the stoma because it is introduced into the ileal segments and covered by the ileostomy bag,
.- it allows selecting patients who are good candidates for closing the ileostomy because of his correct rectoanal function tested preoperatively with a clinical test with the device. With the clinical test the patient may use the defunctionalized part of the bowel and to know how the anal function will be. This selection offers the possibility to exclude for surgery the patients with an improper function. Some actions may be required to correct the defecation disorders previous to surgery in the patients with rectoanal problems detected with the test.
.- it allows checking if there is any leakage or obstruction in the distal bowel before the surgery. With the installation of contrast solutions we can offer a radiologic study of the distal bowel in order to asses any problem.
- it improves the condition of the defunct bowel by preparing it for subsequent transit reconstruction surgery. In this way, a faster functional recovery of the defunct segment is achieved.
.- it improves the function of the distal bowel. The distal part of the ileum and the defunctionalized colon and rectum usually have a glandular atrophy due to the absence of alimentary transit. With the irrigation of some solutions trough the device it is possible to improve the function of the ileum and colon with the pre-habilitation of the glandular tissue.

Other preferred embodiments of the present invention are described in the appended dependent claims and the multiple combinations of those claims.

## Claims

1. Continuity device for derivative ileostomy, comprising:
a collector tube suitable to be inserted inside a first bowel segment of an ileostomy,
a distal tube suitable to be inserted inside a second bowel segment of an ileostomy,
a connector tube having a curved configuration, and made of a deformable material,
wherein the connection tube can be connected and disconnected with the collector and distal tubes, such that, when the connector tube is connected fecal material can circulate through the collector, connection and distal tubes,
the device further comprising a first and second inflatable bags respectively mounted on the collector tube and the distal tube, wherein the inflatable bags are configured to retain the respective tubes fixed to a part of the bowel segments at which are inserted when they are inflated.

2. Device according to claim 1 further comprising first and second conduits extending respectively along the collector and distal tubes, and communicating respectively with the interior of the first and second bags, and wherein the first and second conduits are accessible from outside the device for inflating the bags.

3. Device according to claim 1 or 2, further comprising first and second tubular connection elements for connecting the connection tube with the collector and distal tubes.

4. Device according to claim 3, wherein each connection elements has one end connectable with one end of the connection tube and another end connectable with a proximal end of the collector tube or the distal tube, the connection element having a gripping wall transversally arranged and placed between the two ends of the connection element.

5. Device according to any of the preceding claims further comprising a first frusto-conical funnel coupled at a distal end of the collection tube, and wherein the funnel is preferably made of silicone.

6. Device according to any of the preceding claims, further comprising a second frusto-conical funnel coupled at a distal end of the distal tube, and wherein the funnel is preferably made of silicone.

7. Device according to any of the claims 5 or 6, wherein a part of the inflatable bag is retained between the collection tube or the distal tube and the frusto-conical funnel.

8. Device according to any of the preceding claims, wherein the connection tube is U-shaped.

9. Device according to any of the preceding claims, wherein the connection tube is made of the elastic material, preferable it is made of silicone.

10. Device according to claim 2, wherein the first and second conduits are joined respectively to an inner surface of the collection and distal tubes, or they are integrally formed with the collection and distal tubes.

11. Device according to any of the preceding claims, further comprising first and second valves externally placed to the device, and connected respectively with the first and second conduits, and having an opening for their connection with pumping means.

12. Device according to any of the preceding claims, further comprising a third conduit fluidly communicated with the connection tube interior and accessible from outside the connection tube.
